# EUROPEAN PATENT SPECIFICATION

(11) **EP 3 028 052 B1**
(45) Date of publication and mention of the grant of the patent: **01.11.2017**
(21) Application number: 14748175.8
(22) Date of filing: 01.08.2014
(51) Int. Cl.: G01N 33/86, C12N 5/00

(54) **METHOD FOR PREDICTING TREATMENT RESPONSE AND TEST FOR SAFE USE OF MESENCHYMAL STEM CELLS ON INFLAMMATORY DISEASES.**
VERFAHREN ZUR VORHERSAGE DER BEHANDLUNGSREAKTION UND TEST ZUR SICHEREN ANWENDUNG VON MESENCHYMALEN STAMMZELLEN BEI ENTZÜNDUNGSKRANKHEITEN
MÉTHODE PERMETTANT DE PRÉDIRE LA RÉPONSE À UN TRAITEMENT ET ESSAI PERMETTANT D'UTILISER EN TOUTE SÉCURITÉ DES CELLULES SOUCHES MÉSENCHYMATEUSES EN CAS DE MALADIE INFLAMMATOIRE

(30) Priority: 01.08.2013 EP 13382315
(43) Date of publication of application: 08.06.2016
(73) Proprietor: Fundación Pública Andaluza Progreso Y Salud, 41092 Sevilla (ES); Instituto de Salud Carlos III, 28029 Madrid (ES)
(72) Inventor: SORIA ESCOMS, Bernat, 41092 Sevilla (ES); HMADCHA, Abdelkrim, 41092 Sevilla (ES); ACOSTA LÓPEZ, Lourdes, 41092 Sevilla (ES); ESCACENA ACOSTA, Natalia, 41092 Sevilla (ES)
(74) Representative: Hoffmann Eitle
(86) International application number: PCT/EP2014/066600
(87) International publication number: WO 2015/014988

(56) References cited:
- WO-A1-2012/123401
- WO-A2-00/65349
- WO-A2-03/082072
- WO-A2-2010/017216
- US-A- 5 352 583
- PIERRE THEUMA ET AL: "Inflammation, Insulin Resistance, and Atherosclerosis", METABOLIC SYNDROME AND RELATED DISORDERS, vol. 2, no. 2, 1 June 2004 (2004-06-01), pages 105-113, XP55159102, US ISSN: 1540-4196, DOI: 10.1089/met.2004.2.105
- BIJU PAREKKADANM ARNO W TILLES AND: "Bone marrow-derived mesenchymal stem cells ameliorate autoimmune enteropathy independently of regulatory T cells", STEM CELLS, ALPHAMED PRESS, DAYTON, OH, US, vol. 26, no. 7, 17 April 2008 (2008-04-17) , pages 1913-1919, XP008145390, ISSN: 1066-5099, DOI: 10.1634/STEMCELLS.2007-0790
- OLGA DELAROSA ET AL: "Mesenchymal stem cells as therapeutic agents of inflammatory and autoimmune diseases", CURRENT OPINION IN BIOTECHNOLOGY, vol. 23, no. 6, 1 December 2012 (2012-12-01), pages 978-983, XP55096416, ISSN: 0958-1669, DOI: 10.1016/j.copbio.2012.05.005

## Description

### FIELD OF THE INVENTION

The invention relates to the field of medical prognosis, more specifically to the prognosis of patients with inflammatory diseases treated with isolated mesenchymal stem cells compositions.

### BACKGROUND OF THE INVENTION

Wound healing is a complex process that involves the interaction of inflammation, reepithelialisation, angiogenesis, extracellular matrix deposition and remodelling. This process is essential to maintain the integrity of the normal tissue. In individuals with diabetes mellitus, wound remains in a chronic inflammatory state and fails to heal in a timely an orderly manner. Stem cells have been shown to mobilize and home ischemic and wounded tissue where they secrete chemokines and growth factors that promote angiogenesis and extracellular matrix remodelling, creating a local environment conductive to wound healing (Wu et al., 2007. Stem Cells 25: 2648-59). Stem-cell therapy for the treatment of diabetic non-revascularisable PAD (*peripheral arterial disease*) is an approach with encouraging preliminary results (Ruiz-Salmeron et al., 2011. Cell Transplant. 20: 1629-39). In diabetic patients with critical limb ischemia (CLI), intra-arterial perfusion of bone marrow mononuclear cells is a safe procedure that generates a significant increase in the vascular network in ischemic areas and promotes remarkable clinical improvement (Ruiz-Salmeron et al., 2011. Cell Transplant. 20: 1629-39). Furthermore it has been reported that AdMSCs can be autologously transplanted directly to the wound, promoting enhanced healing of diabetic wounds (Blumberg et al., 2012. Diabetes Res Clin Pract 96: 1-9), contributing to blood vessel formation and cutaneous regeneration. AdMSC release multiple growth factors, including vascular endothelial growth factor (VEGF), hepatocyte growth factor (HGF), and fibroblast growth factor-2 (FGF-2), promoting therapeutic angiogenesis in ischemic tissues (Kilroy et al., 2007. J Cell Physiol 212: 702-9; Kim et al., 2007. J Dermatol Sci 48: 15-24; Nakagami et al., 2005. Arterioscler Thromb Vasc Biol 25: 2542-47). Even more important mesenchymal stromal cells display anti-thrombogenic and anti-inflammatory properties (Hashi et al., 2007. PNAS 104: 11915-20; Prockop et al., 2012. Mol Ther 20: 14-20). Although the homing mechanisms of MSCs are not well understood, the anti-inflammatory and antithrombogenic properties of MSCs prompted parenteral or local use of these cells to promote wound healing and regeneration of damaged tissues. In this context the authors of the present invention and collaborators have conducted a clinical study to evaluate the safety and feasibility of intra-arterial administration of autologous AdMSCs (adipose derived mesenchymal stem cells) in 36 diabetic patients with CLI. Unfortunately, two patients developed an adverse event consisting in appearance of distal micro-thrombosis that could be controlled with an aggressive anti-thrombotic therapy. In contrast the authors of the present invention did not observed any adverse reaction when autologous AdMSCs from non-diabetic patients where used under same conditions. The observed adverse reaction contradicts the described fibrinolytic properties of MSCs (Hashi et al., 2007. PNAS 104: 11915-20; Prockop et al., 2012. Mol Ther 20: 14-20; Neuss et al., 2010. Cells Tissues Organs 191: 36-48). Consequently, there is currently a need to provide a safety test, method and kit, in order to determine the response to the treatment with MSCs of a human subject suffering from inflammatory disease and allocating the human subject in one of two groups, wherein group 1 comprises subjects with low risk of having a thrombotic event after treatment; and wherein group 2 represents the remaining subjects.

### BRIEF DESCRIPTION OF THE INVENTION

A first aspect of the present invention refers to the use of tissue type plasminogen activator (tPA) and/or plasminogen activator inhibitor (PAI-1) for prognosticating or predicting a thrombotic event associated to the treatment with MSCs of a human subject suffering from an inflammatory disease.

A second aspect of the present invention refers to a method for prognosticating or predicting a thrombotic event associated to the treatment with MSCs of a human subject suffering from an inflammatory disease, by predicting or prognosticating the response of said human subject to said treatment, wherein said method comprises:
a. obtaining a sample of isolated MSCs;
b. contacting the isolated MSCs of step (a) with human blood serum obtained by centrifugation of human blood from the human subject,
c. using, as an indicator, expression levels of tissue type plasminogen activator (tPA) protein or of mRNA encoding tPA, in the biological sample obtained from step (b),
wherein the result is indicative of a positive response if the expression levels of protein tPA, or of mRNA encoding tPA, in said biological sample are increased in comparison to a reference sample and/or a positive control.

In a preferred embodiment of the second aspect of the invention increased expression is defined as a level of expression greater than the expression produced in a sample of reference, preferably MSCs in normal culture conditions sample. In a more preferred embodiment of the invention increased is defined as a level of expression greater than or equal to two fold, preferably three fold, of the expression of protein tPA or mRNA encoding tPA in MSCs in normal culture conditions.

A third aspect of the invention refers to a method for prognosticating or predicting a thrombotic event to the treatment with MSCs of a human subject suffering from inflammatory disease, by predicting or prognosticating the response of said human subject to said treatment, wherein said method comprises:
a. obtaining a sample of isolated MSCs,
b. contacting the isolated MSCs from step (a) with human blood serum obtained by centrifugation of human blood from the human subject,
c. using, as an indicator, expression levels of plasminogen activator inhibitor (PAI-1) protein, or mRNA encoding PAI-1, in the biological sample obtained from step (b),
wherein the result is indicative of a positive response if the expression levels of PAI-1, or mRNA encoding PAI-1, are decreased in comparison to a reference sample and/or a positive control.

In a preferred embodiment of the third aspect of the invention decreased expression is defined as a level of expression lower than the expression produced in a sample of reference, preferably MSCs in normal culture conditions sample. In a more preferred embodiment of the invention decreased expression is defined as a level of expression lower than or equal to 1/2, preferably lower than or equal to 1/3, of the expression of protein PAI-1 or mRNA encoding PAI-1, in MSCs in normal culture conditions.

A fourth aspect of the invention refers to a method for prognosticating or predicting a thrombotic event associated to the treatment with MSCs of a human subject suffering from inflammatory disease, by predicting or prognosticating the response of said human subject to said treatment, wherein said method comprises:
a. obtaining a sample of isolated MSCs,
b. contacting the isolated MSCs from step (a) with human blood serum obtained by centrifugation of human blood from the human subject,
c. using, as an indicator, expression levels of tissue type plasminogen activator (tPA) protein or of mRNA encoding tPA, and plasminogen activator inhibitor (PAI-1) protein, or mRNA encoding PAI-1, in the biological sample obtained from step (b),
wherein the result is indicative of a positive response if the expression levels of protein tPA, or of mRNA encoding tPA, in said biological sample are increased in comparison to a reference sample and/or a positive control and if the expression levels of PAI-1, or mRNA encoding PAI-1, are decreased in comparison to a reference sample and/or a positive control.

In a preferred embodiment of the fourth aspect of the invention decreased expression is defined as a level of expression lower than the expression produced in a sample of reference, preferably MSCs in normal culture conditions sample and wherein increased expression is defined as a level of expression higher than the expression produced in a sample of reference, preferably MSCs in normal culture conditions sample. In a more preferred embodiment of the invention, decreased expression is defined as a level of expression lower than or equal to 1/2, preferably lower than or equal to 1/3, of the expression of protein PAI-1 or mRNA encoding PAI-1, in MSCs in normal culture conditions and wherein increased expression is defined as a level of expression greater than or equal to two fold, preferably three fold, of the expression of protein tPA or mRNA encoding tPA in MSCs in normal culture conditions.

In a preferred embodiment of any of the second, third or fourth aspects of the invention the inflammatory disease is selected from the list consisting of: type 2 diabetes, peripheral vascular disease and critical limb ischemia.

A fifth aspect of the invention refers to a method for allocating a human subject suffering from an inflammatory disease in one of two groups, wherein group 1 comprises subjects identifiable by the method according to anyone of the second, third or fourth aspects of the invention as human subjects with positive response; and wherein group 2 represents the remaining subjects. Also disclosed is a pharmaceutical composition comprising isolated MSCs, for treating a human subject of group 1 as identifiable by the method of the fifth aspect of the invention, wherein preferably the pharmaceutical composition also comprises a pharmaceutically acceptable carrier and optionally a further active ingredient.

In a preferred embodiment of the sixth aspect of the invention, the isolated MSCs are human MSCs (hMSCs), more preferably autologous hMSCs and still more preferably bone marrow-derived cultured hMSCs. Also disclosed is a kit suitable for prognosticating or predicting a thrombotic event associated to the treatment with MSCs of a human subject suffering from inflammatory disease, comprising at least one oligonucleotide(s) capable of hybridizing with the mRNAs of any tPA and/or PAI-1. Also disclosed is a kit suitable for prognosticating or predicting a thrombotic event associated to the treatment with MSCs of a human subject suffering from inflammatory disease, which comprises a media having at least a capture antibody capable of complexing with any of biomarker proteins tPA or PAI-1 or a fragment thereof and an assay for the detection of a complex of the biomarker and the capture antibody.

### BRIEF DESCRIPTION OF THE DRAWINGS

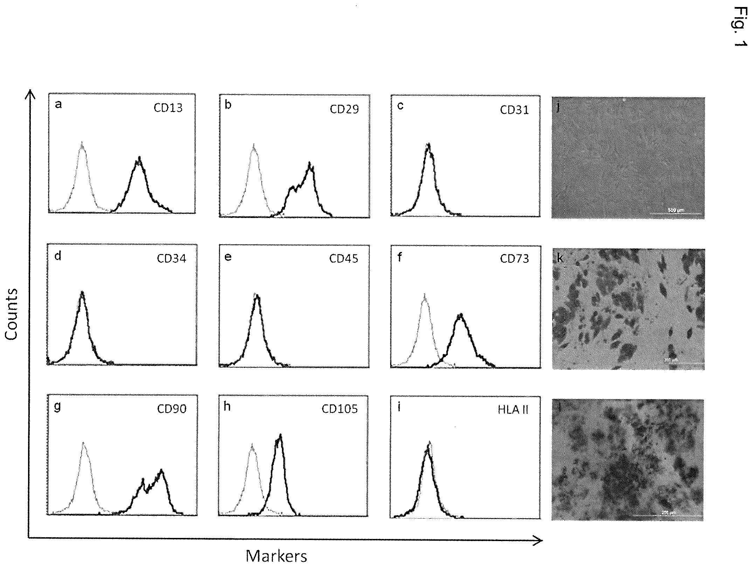

**Fig. 1****:** Characterization of AdMSCs. (a-i) Flow cytometry analysis of cell surface markers of AdMSCs, representative histograms (black line), and their respective controls (gray line). (j) Morphology of AdMSCs populations, displayed a spindle-shaped morphology; (k) Adipogenesis differentiation, detected by the formation of intracytoplasmic lipid droplets stained with oil red O; (I) Osteogenic differentiation was demonstrated by calcium deposition shown by staining with Alizarin red.

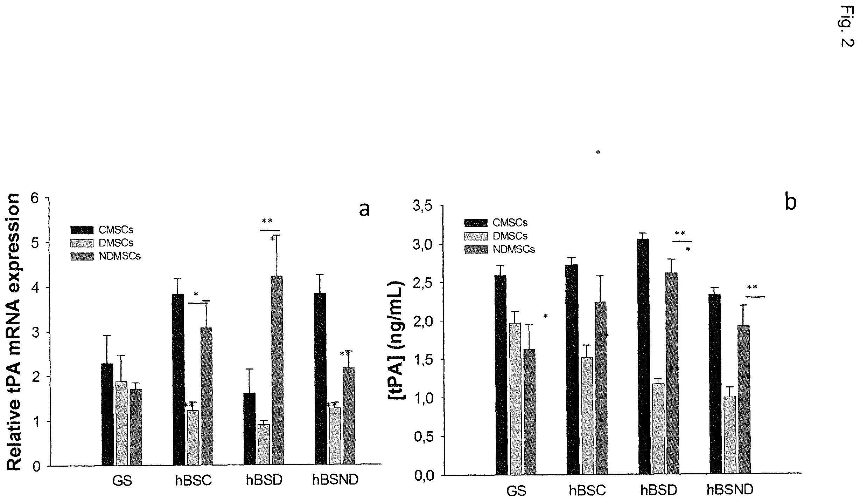

**Fig. 2****:** Detection of tPA mRNA expression (a) and protein release (b) in AdMSCs from human control (AdMSCs), diabetic CLI patient (AdMSC-D) and non-diabetic CLI patient (AdMSC-ND) after stimulation with growth supplement (Control), blood serum from human control (hBS), diabetic CLI patient (hBS-D), and non-diabetic CLI patient (hBSND). Relative mRNA expression indicates the ratio between specific gene and housekeeping genes. Values are normalized to the expression in no stimulated AdMSCs, which was arbitrarily set to 1. Data are represented as mean values ± SEM. n=4. *p≤0·05; **p≤0·01.

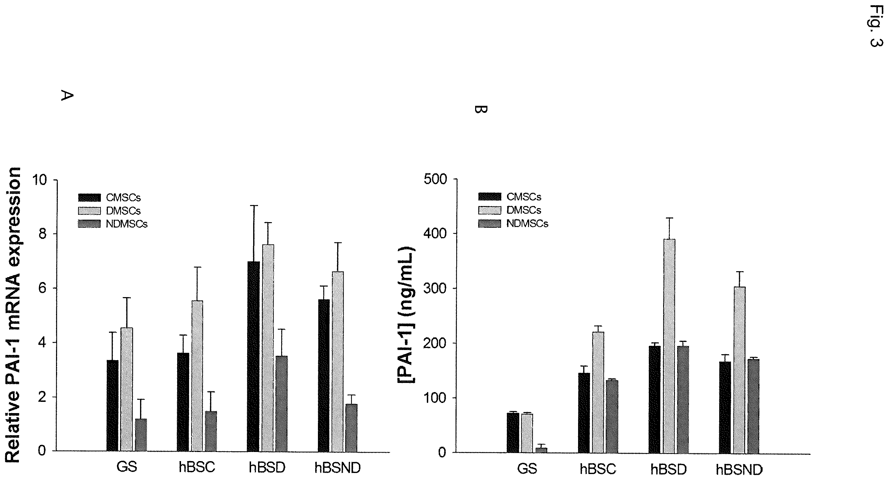

**Fig. 3****:** Detection of PAI-1 mRNA expression (a) and protein release (b) in AdMSCs from human control (AdMSCs), diabetic CLI patient (AdMSC-D), and non-diabetic CLI patient (AdMSC-ND) after stimulation with growth supplement (Control), blood serum from human control (hBS), diabetic CLI patient (hBS-D), and non-diabetic CLI patient (hBS-ND). Relative mRNA expression indicates the ratio between specific gene and housekeeping genes. Values are normalized to the expression in no stimulated AdMSCs, which was arbitrarily set to 1. Data are represented as mean values ± SEM. n=4. *p≤0·05; **p≤0.01.

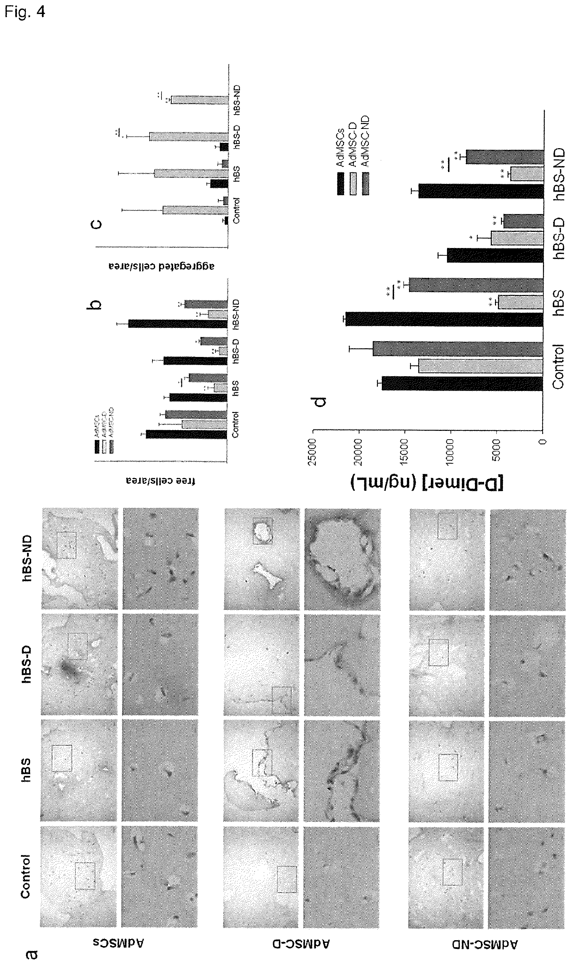

**Fig. 4****:** AdMSCs cultured in fibrin clots, after 24h hMSC-mediated lysis of the fibrin clot became obvious in that a clearing zone surrounded each cell (a). Free cells/area (b) and aggregates/area (c) quantification shows less AdMSC-D free cells and higher AdMSCD cell aggregates. Detection of (D-dimers) a fibrin degradation product (d) in AdMSCs from human control (AdMSCs), diabetic CLI patient (AdMSC-D) and non-diabetic CLI patient (AdMSC-ND) after stimulation of fibrin clots with growth supplement (Control), blood serum from human control (hBS), diabetic CLI patient (hBS-D), and non-diabetic CLI patient (hBS-ND). Data are represented as mean values ± SEM. n=4. *p≤0·05; **p≤0·01.

### DETAILED DESCRIPTION OF THE INVENTION

The present invention provides new methods of using proteins markers tPA and/or PAI-1, or mRNA encoding these proteins, as indicators for good response vs. a response with high probability of suffering a thrombotic event after treatment of a human subject suffering from an inflammatory disease with mesenchymal stem cells.

In order to assess the molecular mechanism underlying clot formation and lysis in patients receiving a treatment with MSCs (mesenchymal stem cells), the inventors focused on key factors of the fibrinolytic cascade (tPA and PAI-1) after the stimulation of AdMSCs (adiposed derived mesenchymal stem cells) with blood serum from patients with CLI either diabetic or non-diabetic. The cellular reactions and interactions of AdMSCs with the fibrin matrix and their fibrinolytic capacity were also studied.

From the results shown in Figure 1 to 4, it can be clearly concluded that increased expression levels of protein tPA, or of mRNA encoding tPA (tissue type plasminogen activator) in comparison to a reference sample and/or a positive control, and/or decreased levels of protein PAI-1 or of mRNA encoding PAI-1 in comparison to a reference sample and/or a positive control, significantly correlate with a better rate of response, namely a reduced risk of suffering a thrombotic event after treatment with MSCs. These results clearly demonstrate the usefulness of these markers, tPA and/or PAI-1, as indicators for survival.

Thus, a first aspect of the invention refers to the use of tissue type plasminogen activator (tPA) and/or plasminogen activator inhibitor (PAI-1), for prognosticating or predicting the response of a thrombotic event associated to the treatment with MSCs of a human subject suffering from inflammatory disease. In a preferred embodiment, tPA and PAI-1 are used simultaneously.

It is noted that tissue type plasminogen activator (tPA; PLAT; TPA; alteplase; plasminogen activator, tissue type; reteplase; t-plasminogen activator; tissue plasminogen activator (t-PA); plasminogen activator, tissue) is located in Chromosome 8 (8p12). This gene encodes tissue-type plasminogen activator, a secreted serine protease that converts the pro-enzyme plasminogen to plasmin, a fibrinolytic enzyme. Tissue-type plasminogen activator is synthesized as a single chain which is cleaved by plasmin to a two chain disulfide linked protein. This enzyme plays a role in cell migration and tissue remodeling. Alternative splicing of this gene results in multiple transcript variants encoding different isoforms. (Genbank accession number NP_127509.1, NM_033011.2)

In the context of the present invention the term "*Plasminogen activator inhibitor-1*" (from herein after referred to as PAI-1) also known as endothelial plasminogen activator inhibitor or serpin E1 is a protein that in humans is encoded by the SERPINE1 gene. PAI-1 is a serine protease inhibitor (serpin) that functions as the principal inhibitor of tissue plasminogen activator (tPA) and urokinase (uPA), the activators of plasminogen and hence fibrinolysis (the physiological breakdown of blood clots). It is a serine protease inhibitor (serpin) protein (SERPINE1).

In the context of the present invention, a "thrombotic event" is defined as the formation or presence of a blood clot in a blood vessel. The vessel may be any vein or artery as, for example, in a deep vein thrombosis or a coronary (artery) thrombosis. The clot itself is termed a thrombus. If the clot breaks loose and travels through the bloodstream, it is a thromboembolism. Thrombosis, thrombus, and the prefix thrombo- all come from the Greek thrombos meaning a lump or clump, or a curd or clot of milk.

In the context of the present invention, inflammation is the final common pathway of various insults, such as infection, trauma, and allergies to the human body. It is characterized by activation of the immune system with recruitment of inflammatory cells, production of pro- inflammatory cells and production of pro-inflammatory cytokines. Most inflammatory diseases and disorders are characterized by abnormal accumulation of inflammatory cells including monocytes/macrophages, granulocytes, plasma cells, lymphocytes and platelets. Along with tissue endothelial cells and fibroblasts, these inflammatory cells release a complex array of lipids, growth factors, cytokines and destructive enzymes that cause local tissue damage.

The inflammatory diseases encompassed by the methods of this invention can stem from a wide range of medical conditions that cause inflammation. Inflammation refers to all phases of the process of inflammation, including the initial signaling events, antigen presentation, dendritic cell activation, antibody production, T-cell activation, B-cell activation and cytokine expression. One type of inflammatory diseases are immunoinflammatory diseases. Examples of immunoinflammatory diseases include such conditions as rheumatoid arthritis, juvenile rheumatoid arthritis, osteoarthritis, transplant rejection, sepsis, acute respiratory distress syndrome, asthma, and cancer. Another type of inflammatory disease are autoimmune diseases. Examples of autoimmune diseases include such conditions as multiple sclerosis, psoriasis, inflammatory bowel disease, glomerulonephritis, lupus, uveitis, and chronic hepatitis. Other inflammatory diseases, including such conditions caused by trauma, oxidative stress, cell death, irradiation damage, ischemic, reperfusion, cancer, transplant rejection, and viral infection, can be the object of the present invention.

Inflammatory diseases of arteries may also cause peripheral vascular disease, wherein in a preferred embodiment of the present invention, the inflammatory disease is the peripheral vascular disease.

The more common risk factors for the development of peripheral vascular disease are diabetes mellitus, hypertension and cigarette smoking as well as a family history of hyperlipidemia. There are two broad categories in which patients with peripheral vascular disease present:

In the context of the present invention, critical limb ischemia (CLI) is a severe blockage in the arteries of the lower extremities, which markedly reduces blood-flow. It is a serious form of peripheral arterial disease. CLI is a chronic condition that results in severe pain in the feet or toes, even while resting. Complications of poor circulation can include sores and wounds that won't heal in the legs and feet. Left untreated, the complications of CLI will result in amputation of the affected limb.

In a preferred embodiment of the present invention, the inflammatory disease is the critical limb ischemia (CLI).

In the context of the present invention, the term "adipose tissue derived mesenchymal cells" or "AdMSCs", as used herein, refers to cells that originate from adipose tissue and are phenotypically characterized in that they are (i) negative for at least one, two, three, four, five, six, seven, eight, nine, ten or preferably all of the following markers CD3, CDI Ib, CD14, CD19, CD31, CD34, CD45, CD62L, CD95L, CDI 17, and HLA-DR cell surface markers, and (ii) positive for at least one, two, three, four, five, six, seven, eight or preferably all of the following markers CD 13, CD29, CD44, CD49e, CD73, CD90, CD 105, CD 166, and HLA-ABC cell surface markers. By "adipose tissue" is meant any fat tissue. The adipose tissue may be brown or white adipose tissue, derived from subcutaneous, omental/visceral, mammary, gonadal, or other adipose tissue site, e.g., the adipose tissue is subcutaneous white adipose tissue.

As used herein, the terms "treat", "treatment" and "treating" refer to the amelioration, cure or remedy of inflammatory disease, which results from the administration of the pharmaceutical composition provided by the present invention comprising a population of MSCs to a subject in need of said treatment. A second aspect of the invention refers to a method for prognosticating or predicting a thrombotic event associated to the treatment with MSCs of a human subject suffering from an inflammatory disease, comprising but not restricted to diabetes type 2, peripheral vascular disease (PVD) and/or critical limb ischemia, by predicting or prognosticating the response of said human subject to said treatment, wherein said method comprises:
(a) obtaining a sample of isolated MSCs, particularly MSCs from adipose tissue, ,
(b) contacting the isolated MSCs from step (a) with human blood serum obtained by centrifugation of human blood from the human subject,
(c) using, as an indicator, expression levels of tissue type plasminogen activator (tPA) protein or of mRNA encoding tPA, in the biological sample obtained in step b.,
wherein the result is indicative of a positive response if the expression levels of protein tPA, or of mRNA encoding tPA, in said biological sample are increased in comparison to a reference sample and/or a positive control.

In a preferred embodiment of the second aspect of the invention, increased expression is defined as a level of expression greater than the expression produced in a sample of reference, preferably MSCs in normal culture conditions sample. In a more preferred embodiment of the invention, increased is defined as a level of expression greater than or equal to two fold, particularly greater than three fold, of the expression of protein tPA or mRNA encoding tPA, in MSCs in normal culture conditions. Please note that the expression of protein tPA in normal culture conditions is understood to be from 2 to 3 ng/ml.

A third aspect of the invention refers to a method for prognosticating or predicting a thrombotic event to the treatment with MSCs of a human subject suffering from inflammatory disease, comprising but not restricted to diabetes type 2, peripheral vascular disease (PVD) and/or critical limb ischemia, by predicting or prognosticating the response of said human subject to said treatment, wherein said method comprises:
(a) obtaining a sample of isolated MSCs, particularly MSCs from adipose tissue,
(b) contacting the isolated AdMSCs from step (a) with human blood serum obtained by centrifugation of human blood from the human subject,
(c) using, as an indicator, expression levels of plasminogen activator inhibitor (PAI-1) protein, or mRNA encoding PAI-1, in the biological sample obtained in step b,
wherein the result is indicative of a positive response if the expression levels of PAI-1, or mRNA encoding PAI-1, are decreased in comparison to a reference sample and/or a positive control.

In a preferred embodiment of the third aspect of the invention, decreased expression is defined as a level of expression lower than the expression produced in a sample of reference, preferably MSCs in normal culture conditions sample. In a more preferred embodiment of the invention, decreased expression is defined as a level of expression lower than or equal to 1/2, particularly lower than or equal to 1/3, of the expression of protein PAI-1 or mRNA encoding PAI-1, in MSCs in normal culture conditions. Please note that the expression of protein PAI-1 in normal culture conditions is understood to be from 150 to 200 ng/ml.

In the context of the present invention, the subjects the response of which is predicted are human subjects suffering from an inflammatory disease. The terms "human subject" "subject" and "patient" are therefore used interchangeably in this specification.

In the context of the present invention "Response" refers to the clinical outcome of the subject. In this sense, a positive response refers to the progression free of a subject from suffering a thrombotic event associated to the treatment with MSCs of a human subject suffering from an inflammatory disease after said subject has undergone treatment.

In the context of the present invention the method of determining the response, i.e. the expression level of the mRNA of tPA or PAI-1, need not be particularly limited, and may be selected from a gene profiling method, such as a microarray, and/or a method comprising PCR, such as real time PCR; and/or Northern Blot or by using immunohistochemistry.

Real time quantitative PCR (RQ-PCR) is a sensitive and reproducible gene expression quantification technique which can particularly be used to profile mRNA expression in cells and tissues. Any method for evaluation of RT-PCR results may be used, and and the ΔΔCt-method may be preferred (Livak et al. Methods 2001, 25:402-408.) (Ct = Cycle threshold values). The ΔΔCt-method will involve a control sample and a treatment sample. For each sample, a target gene and an endogenous control (as described below) gene are included for PCR amplification from (typically serially diluted) aliquots. Typically several replicates are used for each diluted concentration to derive amplification efficiency. PCR amplification efficiency can be defined as percentage amplification (from 0 to 1). During the PPCR reaction, a software typically measures for each sample the cycle number at which the fluorescence (indicator of PCR amplification) crosses an arbitrary line, the threshold. This crossing point is the Ct value. More dilute samples will cross at later Ct values. To quantify mRNA gene expression, the Ct for an RNA or DNA from the mRNA gene of interest is divided by Ct of nucleic acid from the endogenous control, such as non-tumoral tissue, to normalize for variation in the amount and quality of RNA between different samples. This normalization procedure is commonly called the ΔΔCt-method (Schefe et al., 2006, J. Mol. Med. 84: 901-10). ΔΔCt calculations express data in the context of test sample (here: mRNA) versus calibrator (endogenous control). If the ΔΔCt calculation is positive (for example +2.0), then: 2-ΔΔCt = 2-(2.0) = 0.25. The amount of target, normalized to an endogenous reference and relative to a calibrator, is given by: 2-ΔΔCt. Details of the ΔΔCt calculation method can be found in: Applied Biosystems user Bulletin No. 2 (P/N 4303859).

Without prejudice of the method used to determine the response (RQ-PCR, immunohistochemistry etc...), in the context of the present invention an increased expression or a reduced expression are defined in comparison to a normal sample/sample of reference and/or to a positive control.

In this sense, a "normal sample" or a "sample of reference" is defined as a sample that expresses proteins tPA and/or PAI-1 or mRNA encoding any of these proteins, i.e. a sample originating from the same tissue of the biopsy of origin (in this case AdMSCs isolated from adipose tissue of human subjects the control sample would be from human subjects without an inflammatory disease).

In the context of the present invention a positive control sample are normal human cultures of MSCs.

A fourth aspect of the present invention refers to a method for prognosticating or predicting a thrombotic event associated to the treatment with MSCs of a human subject suffering from inflammatory disease, by predicting or prognosticating the response of said human subject to said treatment, wherein said method comprises:
(a) obtaining a sample of isolated MSCs, particularly MSCs from adipose tissue, ,
(b) contacting the isolated MSCs from step (a) with human blood serum obtained by centrifugation of human blood from the human subject,
(c) using, as an indicator, expression levels of tissue type plasminogen activator (tPA) protein or of mRNA encoding tPA, **and** plasminogen activator inhibitor (PAI-1) protein, or mRNA encoding PAI-1, in the biological sample obtained in step b.,;
wherein the result is indicative of a positive response if the expression levels of protein tPA, or of mRNA encoding tPA, in said biological sample are increased in comparison to a reference sample and/or a positive control and if the expression levels of PAI-1, or mRNA encoding PAI-1, are decreased in comparison to a reference sample and/or a positive control.
In a preferred embodiment of any one of the preceding aspects of the invention, the human subject is suffering from diabetes type 2. In a more preferred embodiment of the invention, the human subject suffers from peripheral vascular disease (PVD). In a still more preferred embodiment of the present invention, the human subject is suffering from critical limb ischemia (CLI).
A fifth aspect of the invention refers to a method for allocating a human subject suffering from an inflammatory disease in one of two groups, wherein group 1 comprises subjects identifiable by the method according to the second,third or fourth aspects of the invention as human subjects with positive response; and wherein group 2 represents the remaining subjects.
In a particular aspect of the invention, a human subject with high risk of suffering a thrombotic even of group 2, as identifiable by the method of the fiftth aspect of the invention, might be eligible to a change of treatment. The treatment of choice depends on many different factors such as the type inflammatory disease. Also disclosed is a pharmaceutical composition comprising MSCs and/or other compound for treating a human subject of group 1 as identifiable by the method of the fifth aspect of the invention.

In a preferred embodiment the pharmaceutical composition for the treatment of choice of a human subject of group 1 suffering from an inflammatory disease, as identifiable by the method of the fifth aspect of the invention, comprises isolated MSCs. In a more preferred embodiment of the invention, the MSCs are human autologous MSCs. In a still more preferred embodiment of the present invention, the MSCs are autologous bone marrow-derived cultured MSCs or adipose derived mesenchymal stem cells (AdMSCs).
In the context of the present invention, the term "isolated" applied to a cell population refers to a cell population, isolated from the human or animal body, which is substantially free of one or more cell populations that are associated with said cell population *in vivo* or *in vitro.*

The pharmaceutical composition here disclosed is a cell-containing pharmaceutical composition comprising MSCs. This cell-containing composition is not particularly limited in terms of other arrangements such as compositional arrangement (buffer liquid, culture liquid, or the like), cell number, etc. Moreover, the cell-containing pharmaceutical composition according to the present invention preferably contains a secreta (e.g., growth factor, or the like) secreted from cells contained in the cell-containing pharmaceutical composition.
The cell-containing composition can be used as a drug (pharmaceutical composition) for regenerative medicine. That is, a drug according to the present invention for regenerative medicine is not limited particularly in terms of other specific arrangements, provided that it comprises the cell-containing composition. For example, the use of the drug may be such that the undifferentiated MSCs are differentiated to cells of a kind as suitable for the use, and then used. More specifically, this may be carried out in such a manner that the MSCs are differentiated by using a differentiation inducing material such as a cytokine or the like, and then the differentiated calls are administered to a patient to be treated with the regenerative medicine. Therefore, in a preferred embodiment the present invention encompasses drugs for regenerative medicine containing a cell composition obtained by differentiation of the undifferentiated mesenchymal stem cells, apart from the drugs containing the undifferentiated mesenchymal stem cells.
Moreover, administration conditions of the drug for regenerative medicine in actual clinical applications may be determined as appropriate by animal experiments or the like performed as standard methods in this field. That is, the conditions suitable for prevention or therapeutic effects may be determined via animal experiments to study the administration conditions such as dosage, administration intervals, administration routes, etc. The drug for regenerative medicine can be utilized as a drug for "regenerative medicine by cell transplantation" which regenerates a tissue lost by a disease or impairment and resumes a function, which tissue cannot be regenerated by a conventional therapeutic method.
The drug for regenerative medicine is not limited to treatment of a particular disease, symptom, clinical profile, or the like, provided that the drug is used for the purpose of "regenerative medicine by cell transplantation". More specific examples of the drug for regenerative medicine include transplantation of marrow mesenchymal stem cells to a patient of critical limb ischemia, transplantation of marrow mesenchymal stem cells to a patient of a periodontal disease, transplantation of marrow mesenchymal stem cells to a patient of osteoarthritis, to a patient of diabetes mellitus, and the other transplantation. The drug for regenerative medicine may be used as a composition by mixed with a pharmaceutically allowable carrier. Examples of the carrier encompass sterilized water, physiological saline, buffers, plant oil, emulsifiers, suspending agents, salts, stabilizers, preservatives, surfactants, release controllers, other proteins (BSA etc.), transfection reagents (encompassing lipofection reagents, liposome, and the like), and the like. Moreover, the following carriers are applicable in the present invention: extracellular matrixes such as glucose, lactose, gum Arabic, gelatin, mannitol, starch paste, magnecium trisilicate, talc, corn starch, keratin, colloid silica, potato starch, urea, hyaluronic acid, collagen, etc.; polylactose, calcium phosphate carrier, etc.
The drug may have any form. For example, the drug may have forms of solution (injection-type), microcapsule, tablet, and the like. The drug may be administered systematically or locally. The local administration is preferable if the systematic administration is side effective or is not so effective as the local administration. Moreover, the drug may be administered to a patient in any way and the administration may be, for example, surgical, percutaneous, transbronchial, muscular, interperitoneal, intravenous, intra-arterial, intra-articular, subdermal, medullary, intracerebroventricular, or oral. The drug may be systematically administered or locally. The local administration to lesion section is preferable if the systematic administration is side effective. Dosage and the way of administration may be varied depending on weight, age, and symptom of the patient, therapeutic purpose, and tissue mobility of the active constituents of the drug, and the other factors. A person skilled in the art can arbitrarily select the dosage and the way of administration. Also disclosed is a kit suitable to put into practice the method of the invention, comprising at least one oligonucleotide(s) capable of hybridizing with the mRNAs of either of tPA and/or PAI-1.
The kit is based on the prognostic power of the method of the present invention. In the particular case of the kit, the reference value indicative for non-response (and/or a reference value indicative for response) may be provided with the kit. With the help of the kit, the expression of each target mRNA can be calculated, i.e. relative to, such as the endogenous control samples exemplified above. The endogenous control can thus also be comprised within the kit.

It is preferred that said oligonucleotide(s) hybridizes with two mismatches or less, and preferably with no mismatch, with respect to the mRNA to be determined. As far as hybridization of the oligonucleotide(s) is concerned, it is preferred that said oligonucleotide(s) is capable to do so under stringent conditions. Stringency is a term used in hybridization experiments. Stringency reflects the degree of complementarity between the oligonucleotide and the nucleic acid (which is in this case the mRNA to be detected); the higher the stringency, the higher percent homology between the probe and filter bound nucleic acid. It is well known to the skilled person that the temperature and salt concentrations have a direct effect upon the results that are obtained. It is recognized that the hybridization results are related to the number of degrees below the Tm (melting temperature) of DNA at which the experiment is performed. Often, stringent conditions are defined as a wash with 0.1X SSC (saline-sodium citrate (SSC) buffer at 65°C. (SSC is typically provided as 20X stock solution, which consists of 3 M sodium chloride and 300 mM trisodium citrate (adjusted to pH 7.0 with HCI)).

In particular embodiments, the kit is selected from (a) a kit suitable for PCR, (b) a kit suitable for Northern Blot and (c) a kit suitable for microarray analysis. Any two or more of these embodiments may also be combined, so that the kit may comprise, for example both (a) and (c).

As regards (a) a kit suitable for PCR, this PCR is typically real-time quantitative PCR (RQ-PCR), a sensitive and reproducible gene expression quantification technique.

A Northern Blot involves the use of electrophoresis to separate RNA samples by size and subsequent detection with an oligonucleotide(s) (hybridization probe) complementary to (part of) the target sequence of the RNA of interest.

It is also possible that the oligonucleotide(s) are immobilized in spots on a (preferably solid) surface. In one embodiment thereof, the kit comprises a microarray. An RNA microarray is an array on a solid substrate (usually a glass slide or silicon thin-film cell) that assays large amounts of different RNAs which are detectable via specific probes immobilized on spots on the solid substrate. Each spot contains a specific nucleic acid sequence, typically a DNA sequence, known as probes (or reporters). While the number of spots is not as such limited, there is a preferred embodiment in which the microarray is customized to the methods of the invention. In one embodiment, such a customized microarray comprises fifty spots or less, such as thirty spots or less, including twenty spots or less.

A further embodiment refers to a kit suitable for detecting the level of expression of tPA and/or PAI-1 which comprises a media having affixed thereto a capture antibody capable of complexing with any of biomarker proteins tPA or PAI-1 or a fragment thereof and an assay for the detection of a complex of the biomarker and the capture antibody.
As with the previous kit, this kit is based on the prognostic power of the method of the present invention. In the particular case of the kit, the reference value indicative for non-response (and/or a reference value indicative for response) may be provided with the kit. With the help of the kit, the expression of each target mRNA can be calculated, i.e. relative to, such as the endogenous control samples exemplified above. The endogenous control can thus also be comprised within the kit.
The kit may be used and the use is not particularly limited, although use in the method of the invention in any of its embodiments is preferred.
One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

All publications mentioned in the specification are indicative of the levels of those ordinary skills in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should understood that although the present invention has been specifically disclosed by preferred embodiments and optional features, modification and variation of the concepts herein disclosed may be resorted to by those skilled in the art, and that such modifications and variations to be within the scope of this invention as defined by the appended claims.

The following examples serve to illustrate the present invention; these examples are in no way intended to limit the scope of the invention.

### EXAMPLES

### Materials and methods

### Samples

Abdominal adipose tissue and blood were obtained from diabetic and non-diabetic patients enrolled in two clinical trials (EudraCT: 2008-001387-88 and EudraCT: 2009-013554-32). The donors provided informed consent, and the local and regional medical research ethics committees approved the handling of these samples.

### Cell culture

AdMSCs were isolated from adipose tissue of diabetic and non-diabetic patients with CLI by digestion with collagenase A. Briefly, tissue sample was washed with PBS, homogenized with Ika Ultra Turrax Drive and digested with collagenase A during two hours and a half at 37°C. The digestion was stopped with expansion medium (DMEM supplemented with 10% FBS, 1% Peniciline/Streptomycine, 1% L-Glutamine). After centrifugation, semi-solid phase was filtered with 100µm cell strainer, mixed with the pellet of cells and washed with PBS. The cells were counted in a Neubauer camera and seeded at 1·2-2·0x10⁴cells/cm². After 24h, the medium was changed to wash away any non-adherent cells. Control AdMSCs were obtained from ATCC (ATCC-PCS-500-011). AdMSCs were expanded and maintained in Mesenchymal Stem Cell Basal Medium (MSCBM - Lonza) supplemented with growth supplements (GS: MSCGM™ SingleQuots®, Lonza) and cultured at 37°C in a 20% O₂ and 5% CO₂ humidified atmosphere When 80-90% of confluence is reached, AdMSCs were trypsinized and reseeded in a density of 5x10³ cells/cm² for optimal proliferation.

AdMSCs were characterized by the following specific markers: CD13, CD29, CD31, CD34, CD45, CD73, CD90, HLA-II and CD105 and by differentiation potential after cell expansion. The medium for adipogenic induction used was Adipogenic Induction Medium (Lonza), supplemented with h-Insulin (recombinant), L-Glutamine, Mesenchymal Cell Growth supplement (MCGS), GA-1000, Dexamethasone, Indomethacin and IBMX (Lonza). After 3 cycles of induction/maintenance, cells were fixed with 10% formalin and stained with Oil Red O solution (Sigma). The osteogenic induction medium consisted of Osteogenic Basal Medium (Lonza) supplemented with Dexamethasone, Ascorbate, MCGS, L-Glutamine, Penicillin/Streptomycin, β-glycerophosphate (Lonza). After two weeks in differentiation medium the cells were fixed and stained with Alizarin Red (Sigma).

### Blood samples

Human blood serum was obtained by centrifugation of human blood from diabetic and non-diabetic patients with CLI, and also from healthy volunteer donors. Serum was aliquoted and frozen at -20°C until it posterior use.

### Experimental setups

AdMSCs were plated into culture dishes at 5x10⁴ cells/cm² in MSCBM (Lonza) supplemented with MSCGM™ SingleQuots® (Lonza) and cultured at 37°C in a 20% O₂ and 5% CO₂ humidified atmosphere during 24h. The next 24h MSCs were cultured with serum free MSCBM and the following day with MSCBM supplemented with growth supplements (GS: MSCGM™ Single Quots®, Lonza) or human blood sera. 24h after, the supernatants were collected, aliquoted and frozen for its posterior analysis, and the cells were washed with PBS twice before its collection.

### RNA isolation and cDNA synthesis

AdMSCs total RNA was extracted using Trizol® according to manufacturer's instructions, and the RNA concentration was measured spectrophotometrically (Nanodrop). The remaining genomic DNA was digested with RQ1 RNAse-Free DNAse from bovine pancreas (Promega) by incubation at 37°C for 30 minutes. Reverse transcription was carried out with 2µg of total RNA using M-MLV Reverse Transcriptase (Promega) according to the manufacturer's instructions.

### qRT-PCR

Oligonucleotides primers were designed using the Primer Express version 3.0 software (Applied Biosystems). Each primer pair was selected so that the amplicon spanned an exon junction to preclude amplification of possible genomic DNA. Primers used to amplify PAI-1 transcript were: Forward (5'-CTC TCT CTg CTG CCC TCA CCA AC-3') and Reverse (5'-gTg GTG gAg GAG Agg AGG CTC TTg TTG gTC GTC TgTG-3') and for tPA were: Forward (5'-CAA gTC GTC TCC TTC CCC TTT CC-3') and Reverse (5'-ggg GGG TTg TTG Tgg TGG CAA CAg CAG AAA gTGT-3'). Cyclophilin A (PPIA, tataabiocenter) and 60S acidic ribosomal protein P0 (RPLP, tataabiocenter) were selected as endogenous controls to correct for potential variation in RNA loading or efficiencies of the reverse transcription or amplification reaction.

For relative quantification cDNA sequences were amplified using comparative C_{T} assay (Applied Biosystems). To this end, 10µL SensiFAST SYBR Lo-ROX Mix (including polymerase, dNTPs and buffer) (Bioline), 0·8µL forward primer (400nM), 0·8µL reverse primer (400nM), 10ng cDNA and H₂O to 20µL final volume reaction were assayed in RQ-plates Real-Time PCR System (Applied Byosistems). Thermal cycling conditions were 2 minutes at 95°C followed by 40 cycles of 2-step PCR consisting of 5 seconds at 95°C and 25 seconds at annealing temperature (60°C for PAI-1 and 64°C for tPA). All samples were amplified in triplicate.

### ELI SA

For PAI-1 and tPA proteins detection, ELISA were performed. Supernatants from AdMSCs stimulated or not with growth supplement or human blood serum were transferred to precoated ELISA plates (Bender MedSystems) and proteins were detected according to manufacturer's instructions.

### Fibrin gel culture

Fibrin gels containing AdMSCs were produced as follow: 20µl of thrombin (10units/ml; Sigma) were poured into a 24 well cell culture plate, and 180µl of fibrinogen-cell suspension was added. This suspension consisted of 50µl CaCl₂ (50mM; Sigma), 20µl PBS, 830µl fibrinogen (20mg/ml; Sigma) and 100µl cell suspension (7·5x10⁶ cells/ml). For negative controls, cell suspension was replaced by culture medium. After 15min of polymerization, 1 ml of serum free culture medium (MSCBM) in absence or presence of serum (GS) or human blood sera (hBS, hBS-D, hBS-ND) was added to the clotted fibrin gels. Cells were cultured in a 20% O₂ and 5% CO₂ humidified atmosphere at 37°C. After 24h, medium was removed for D-dimer detection, while the gels were fixed in formalin for histological analysis.

### Histological analysis

Gels containing AdMSCs were fixed in formalin for approximately 24h, dehydrated in a graded ethanol series and embedded in paraffin. Sections of 2µm thick were cut and transferred to microscope slides, dewaxed, rinsed in distilled water and stained with hematoxylin-eosin.

### Cell aggregates analysis

Image analysis freeware (Image J) was used to quantify the number of free and aggregated-cells images are from 4 arbitrary images/slide/condition.

### D-dimer detection

Degradation products of fibrin clots after cell-fibrin construct culture (D-dimers) were detected following the manufacturer's instructions for the Blue D-Dimer kit (Teco). The assay contains antibody coated latex particles. In the presence of D-dimer the particles aggregate to larger aggregates and light scattering decreases. The absorbance measure is proportional to the amount of D-dimer in the sample.

### Statistical analysis

The results were processed for statistical analysis with the SPSS for Windows package (SPSS, Chicago, IL). Unless otherwise stated, data are represented as mean values ± SEM. Data were analyzed with one-way and two-way ANOVA tests. The Tukey test was used for comparisons. Values of p≤0·05 were considered statistically significant.

### Results

Expanded AdMSCs were positive for CD13, CD90, CD105, CD73 (mesenchymal markers) (figure 1 a,b,c,d), and CD29 (adhesion marker) (figure 1e), whereas they were negative for CD34, CD45 (hematopoietic cell markers) (figure 1f,g), CD31 (endothelial cell marker) (figure 1h), and HLA-DR (human leukocyte differentiation antigen class II) (figure 1i). To demonstrate the multipotency of isolated MSCs, cells were differentiated into adipocytes and osteoblasts. After adipogenic and osteogenic induction, the lipid vacuoles of the adipocytes were visualized with Oil Red O (figure 1k) and the mineralized matrix surrounding the osteoblasts with Alizarin Red (figure 1l). Untreated MSCs stained negative both for Oil Red O and Alizarin Red and displayed a spindle-shaped morphology (figure 1j).

The expression and release of key components of the fibrinolytic cascade (tPA, PAI-1) was evaluated after stimulation of control AdMSC, AdMSC from diabetic patients with CLI (AdMSC-D), and non-diabetic patients with CLI (AdMSC-ND) with blood serum from healthy control (hBS), diabetic patients with CLI (hBS-D), and non-diabetic patients with CLI (hBS-ND) such as described in methods. Thereafter, the supernatants were collected for ELISA, and cells were harvested to extract RNA. As PAI-1 has a short span of activity within 0·5-2 hours under physiological conditions,**¹⁶** we conducted time course experiments to check the stability of the protein secreted by AdMSCs in the supernatants after stimulation with different serum; the optimal quantified concentration was detected 24h after stimulation for all conditions assayed (data not show). Figure 2 shows tPA mRNA (a) and protein (b) expression after the stimuli of AdMSCs with GS (Control DESCRIBE), hBS, hBS-D and hBS-ND.

AdMSCs similarly express and release tPA in normal culture conditions (figure 2a, b). When the cells were stimulated with hBS (Human blood serum) we observed that, while for AdMSCs and AdMSC-ND (non-diabetic), the tPA expression was enhanced 3·8 and 3·1-fold, respectively, for AdMSC-D (diabetic) the expression observed was similar to the non-stimulated cells (figure 2a); the protein levels showed the same results (figure 2b). Thus, hBS up-regulated tPA expression in control and AdMSC-ND and did not stimulated the expression of this gene in AdMSC-D. The results observed after stimulation with hBS-D showed an enhancement of 4-fold in the tPA expression of AdMSC-ND, while control and AdMSC-D were not modified (figure 2a). Protein levels showed a minor expression of tPA in AdMSC-D, while AdMSCs expressed levels very similar to the observed after GS (control) and hBS stimulation (figure 2b). The stimulation with hBS-ND gave very similar results as compared with the hBS stimulation assay. So, for all the conditions assayed, AdMSC-D showed a minor expression of tPA in comparison to AdMSCs and AdMSC-ND; moreover, we observed that the stimulated release of tPA from AdMSC-D was always inferior to that of AdMSC and AdMSC-ND.

AdMSCs express PAI-1 in control culture condition although AdMSC-ND expressed minor levels (figure 3). After the stimulation with hBS, expression of PAI-1 was similar to the control at mRNA levels (figure 3a), whilst protein levels displayed an increase in PAI-1 concentration for all the MSCs assayed compared to control (figure 3b). In MSCs from diabetic patients AdMSC-D, the expression of PAI-1 at mRNA levels was higher after the stimulation with hBS-D and hBS-ND (figure 3a); protein levels were similar for AdMSCs and AdMSC-ND after hBS stimulation, whereas AdMSC-D showed an enhanced of 2-fold compared to hBS assay (figure 3b). The response observed after stimulation with hBS-ND was very similar to hBS-D one, although the levels were slightly lower (figure 3). For all the conditions assayed, AdMSC-D showed a higher expression of PAI-1, at mRNA and protein levels, and always higher after the stimulation with hBS-D.

Finally, we embedded AdMSCs intro fibrin clots to mimic an *ex vivo* fibrinolytic situation. After 24h we observed continuous degradation of the fibrin clots, as judged by the increase in clearing zone diameter surrounding cells, except for AdMSC-D (figure 4a) which remarkably showed a different shape consisting in less isolated cells (figure 4b) and high number of aggregated cells per area (figure 4c). To quantify the fibrinolytic activity of these cells, we harvested the supernatants of the fibrin-cells constructs and measured D-dimers, the degradation products of fibrin; as shown in Figure 4d, all AdMSCs used where highly active in cleaving fibrin in control conditions, as shown by the presence of D-dimers in the culture supernatants. For all the conditions assayed, AdMSCs degraded more fibrin than the others cells used; moreover, we observed a minor D-dimer concentration when AdMSCs were stimulated with hBS-D. The pattern observed for AdMSC-ND was very similar to AdMSCs, except when these cells are stimulated with hBS-D where D-dimer concentration was lower. Interestingly, AdMSC-D showed always the lowest concentration of D-dimers, independently of the hBS assayed, and failed to degrade the fibrin clots.

## Claims

1. Use of tissue type plasminogen activator (tPA) and/or plasminogen activator inhibitor (PAI-1), for prognosticating or predicting in vitro a thrombotic event associated to the treatment with mesenchymal stem cells (MSCs) of a human subject suffering from an inflammatory disease.

2. A method for prognosticating or predicting a thrombotic event associated to the treatment with MSCs of a human subject suffering from an inflammatory disease, by predicting or prognosticating the response of said human subject to said treatment, wherein said method comprises:
a. obtaining isolated MSCs from a sample;
b. contacting the isolated MSCs of step (a) with human blood serum obtained by centrifugation of human blood from the human subject,
c. using, as an indicator, expression levels of tissue type plasminogen activator (tPA) protein or of mRNA encoding tPA, in the biological sample obtained from step (b),
wherein the result is indicative of a positive response if the expression levels of protein tPA, or of mRNA encoding tPA, in said biological sample are increased in comparison to a reference sample and/or a positive control.

3. The method of claim 3, wherein increased expression is defined as a level of expression greater than the expression produced in a sample of reference, preferably MSCs in normal culture conditions sample.

4. The method of claim 3, wherein increased is defined as a level of expression greater than or equal to two fold of the expression of protein tPA or mRNA encoding tPA in MSCs in normal culture conditions.

5. A method for prognosticating or predicting a thrombotic event to the treatment with MSCs of a human subject suffering from inflammatory disease, by predicting or prognosticating the response of said human subject to said treatment, wherein said method comprises:
a. obtaining isolated MSCs from a sample,
b. contacting the isolated MSCs from step (a) with human blood serum obtained by centrifugation of human blood from the human subject,
c. using, as an indicator, expression levels of plasminogen activator inhibitor (PAI-1) protein, or mRNA encoding PAI-1, in the biological sample obtained from step (b),
wherein the result is indicative of a positive response if the expression levels of PAI-1, or mRNA encoding PAI-1, are decreased in comparison to a reference sample and/or a positive control.

6. The method of claim 5, wherein decreased expression is defined as a level of expression lower than the expression produced in a sample of reference, preferably MSCs in normal culture conditions sample.

7. The method of claim 5, wherein decreased expression is defined as a level of expression lower than or equal to 1/2, of the expression of protein PAI-1 or mRNA encoding PAI-1, in MSCs in normal culture conditions.

8. A method for prognosticating or predicting a thrombotic event associated to the treatment with MSCs of a human subject suffering from inflammatory disease, by predicting or prognosticating the response of said human subject to said treatment, wherein said method comprises:
a. obtaining isolated MSCs from a sample,
b. contacting the isolated MSCs from step (a) with human blood serum obtained by centrifugation of human blood from the human subject,
c. using, as an indicator, expression levels of tissue type plasminogen activator (tPA) protein or of mRNA encoding tPA, and plasminogen activator inhibitor (PAI-1) protein, or mRNA encoding PAI-1, in the biological sample obtained from step (b);
wherein the result is indicative of a positive response if the expression levels of protein tPA, or of mRNA encoding tPA, in said biological sample are increased in comparison to a reference sample and/or a positive control and if the expression levels of PAI-1, or mRNA encoding PAI-1, are decreased in comparison to a reference sample and/or a positive control.

9. The method of claim 8, wherein decreased expression is defined as a level of expression lower than the expression produced in a sample of reference, preferably MSCs in normal culture conditions sample and wherein increased expression is defined as a level of expression higher than the expression produced in a sample of reference, preferably MSCs in normal culture conditions sample.

10. The method of claim 8, wherein decreased expression is defined as a level of expression lower than or equal to 1/2, of the expression of protein PAI-1 or mRNA encoding PAI-1, in MSCs in normal culture conditions and wherein increased expression is defined as a level of expression greater than or equal to two fold of the expression of protein tPA or mRNA encoding tPA in MSCs in normal culture conditions.

11. The method according to any one of claims 1-10, wherein the inflammatory disease is the diabetes type 2.

12. The method according to any one of claims 1-10, wherein the inflammatory disease is peripheral vascular disease.

13. The method according to any one of the preceding claims, wherein the peripheral vascular disease is a critical limb ischemia.

14. A method for allocating a human subject suffering from an inflammatory disease in one of two groups, wherein group 1 comprises subjects identified by the method according to anyone of claims 1-10 as human subjects with positive response; and wherein group 2 represents the remaining subjects.

## Patentansprüche

1. Verwendung vom gewebespezifischen Plasminogenaktivator (tPA) und/oder Plasminogenaktivatorinhibitor (PAI-1) zur Prognose oder Vorhersage eines thrombotischen Ereignisses in Verbindung mit der Behandlung mit MSCs eines menschlichen Probanden, welcher an einer Entzündungskrankheit leidet.

2. Verfahren zur Prognose oder Vorhersage eines thrombotischen Ereignisses in Verbindung mit der Behandlung mit MSCs eines menschlichen Probanden, welcher an einer Entzündungskrankheit leidet, mittels der Vorhersage oder Prognose der Reaktion des genannten menschlichen Probanden auf die genannte Behandlung, wobei das genannte Verfahren Folgendes umfasst:
a. das Erhalten einer Probe von isolierten MSCs;
b. das Inkontaktbringen der isolierten MSCs aus Schritt (a) mit menschlichem Blutserum, welches durch Zentrifugation des menschlichen Blutes des menschlichen Probanden erhalten wurde,
c. das Verwenden, als Indikator, von Expressionsniveaus des gewebespezifischen Plasminogenaktivator (tPA)-Proteins oder der tPA-kodierenden mRNS, in der aus Schritt (b) erhaltenen biologischen Probe,
wobei das Ergebnis auf eine positive Reaktion hinweist, wenn die Expressionsniveaus des tPA-Proteins, oder der tPA-kodierenden mRNS, in der genannten biologischen Probe im Vergleich zu einer Referenzprobe und/oder einer positiven Kontrolle erhöht sind.

3. Verfahren nach Anspruch 3, wobei eine erhöhte Expression als ein Expressionsniveau definiert wird, welches größer als die in einer Referenzprobe erzeugten Expression, vorzugsweise MSCs in einer Probe unter normalen Kulturbedingungen, ist.

4. Verfahren nach Anspruch 3, wobei erhöht als ein Expressionsniveau definiert wird, welches größer als oder gleich zwei Mal die Expression des tPA-Proteins oder der tPA-kodierenden mRNS in MSCs unter normalen Kulturbedingungen, ist.

5. Verfahren zur Prognose oder Vorhersage eines thrombotischen Ereignisses in Verbindung mit der Behandlung mit MSCs eines menschlichen Probanden, welcher an einer Entzündungskrankheit leidet, mittels der Vorhersage oder Prognose der Reaktion des genannten menschlichen Probanden auf die genannte Behandlung, wobei das genannte Verfahren Folgendes umfasst:
a. das Erhalten einer Probe von isolierten MSCs,
b. das Inkontaktbringen der isolierten MSCs aus Schritt (a) mit menschlichem Blutserum, welches durch Zentrifugation des menschlichen Blutes des menschlichen Probanden erhalten wurde,
c. das Verwenden, als Indikator, von Expressionsniveaus des Plasminogenaktivatorinhibitor (PAI-1)-Proteins, oder der PAI-1-kodierenden mRNS, in der aus Schritt (b) erhaltenen biologischen Probe,
wobei das Ergebnis auf eine positive Reaktion hinweist, wenn die Expressionsniveaus des PAI-1, oder der PAI-1-kodierenden mRNS, im Vergleich zu einer Referenzprobe und/oder einer positiven Kontrolle vermindert sind.

6. Verfahren nach Anspruch 5, wobei eine verminderte Expression als ein Expressionsniveau definiert wird, welches niedriger als die in einer Referenzprobe erzeugten Expression, vorzugsweise MSCs in einer Probe unter normalen Kulturbedingungen, ist.

7. Verfahren nach Anspruch 5, wobei eine verminderte Expression als ein Expressionsniveau definiert wird, welches niedriger als oder gleich 1/2 der Expression des PAI-1-Proteins oder der PAI-1-kodierenden mRNS in MSCs unter normalen Kulturbedingungen ist.

8. Verfahren zur Prognose oder Vorhersage eines thrombotischen Ereignisses in Verbindung mit der Behandlung mit MSCs eines menschlichen Probanden, welcher an einer Entzündungskrankheit leidet, mittels der Vorhersage oder Prognose der Reaktion des genannten menschlichen Probanden auf die genannte Behandlung, wobei das genannte Verfahren Folgendes umfasst:
a. das Erhalten einer Probe von isolierten MSCs,
b. das Inkontaktbringen der isolierten MSCs aus Schritt (a) mit menschlichem Blutserum, welches durch Zentrifugation des menschlichen Blutes des menschlichen Probanden erhalten wurde,
c. das Verwenden, als Indikator, von Expressionsniveaus des gewebespezifischen Plasminogenaktivator (tPA)-Proteins oder der tPA-kodierenden mRNS, und des Plasminogenaktivatorinhibitor (PAI-1)-Proteins, oder der PAI-1-kodierenden mRNS, in der aus Schritt (b) erhaltenen biologischen Probe;
wobei das Ergebnis auf eine positive Reaktion hinweist, wenn die Expressionsniveaus des tPA-Proteins, oder der tPA-kodierenden mRNS, in der genannten biologischen Probe im Vergleich zu einer Referenzprobe und/oder einer positiven Kontrolle erhöht sind und, wenn die Expressionsniveaus des PAI-1, oder der PAI-1-kodierenden mRNS, im Vergleich zu einer Referenzprobe und/oder einer positiven Kontroller vermindert sind.

9. Verfahren nach Anspruch 8, wobei eine verminderte Expression als ein Expressionsniveau definiert wird, welches niedriger als die in einer Referenzprobe erzeugten Expression, vorzugsweise MSCs in einer Probe unter normalen Kulturbedingungen, ist und wobei eine erhöhte Expression als ein Expressionsniveau definiert wird, welches höher als die in einer Referenzprobe erzeugten Expression, vorzugsweise MSCs in einer Probe unter normalen Kulturbedingungen, ist.

10. Verfahren nach Anspruch 8, wobei eine verminderte Expression als ein Expressionsniveau definiert wird, welches niedriger als oder gleich 1/2 der Expression des PAI-1-Proteins oder der PAI-1-kodierenden mRNS in MSCs unter normalen Kulturbedingungen ist und wobei eine erhöhte Expression als ein Expressionsniveau definiert wird, welches größer als oder gleich zwei Mal die Expression des tPA-Proteins oder der tPA-kodierenden mRNS in MSCs unter normalen Kulturbedingungen ist.

11. Verfahren nach einem der Ansprüche 1-10-4, wobei die Entzündungskrankheit Diabetes Typ 2 ist.

12. Verfahren nach einem der Ansprüche 1-10-4, wobei die Entzündungskrankheit eine periphere Gefäßerkrankung ist.

13. Verfahren nach einem der vorhergehenden Ansprüche, wobei die periphere Gefäßerkrankung eine kritische Extremitätenischämie ist.

14. Verfahren zur Zuweisung eines menschlichen Probanden, welcher an einer Entzündungskrankheit leidet, zu einer von zwei Gruppen, wobei Gruppe 1 Probanden umfasst, welche mittels des Verfahrens nach einem der Ansprüche 1-10 als menschliche Probanden mit positiver Reaktion identifiziert werden können; und wobei Gruppe 2 die restlichen Probanden darstellt.

## Revendications

1. Utilisation de l'activateur tissulaire du plasminogène (tPA) et/ou d'un inhibiteur de l'activateur du plasminogène (PAI-1) permettant de pronostiquer ou prédire un événement thrombotique associé au traitement avec des CSM d'un sujet humain souffrant d'une maladie inflammatoire.

2. Méthode permettant de pronostiquer ou prédire un événement thrombotique associé au traitement avec des CSM d'un sujet humain souffrant d'une maladie inflammatoire, en prédisant ou pronostiquant la réponse dudit sujet humain audit traitement, dans laquelle ladite méthode comprend :
a) l'obtention d'un échantillon de CSM isolées,
b) le contact des CSM isolées de l'étape (a) avec du sérum sanguin humain obtenu par centrifugation de sang humain du sujet humain,
c) utilisation, comme indicateur, de niveaux d'expression de protéine de l'activateur tissulaire du plasminogène (tPA) ou d'ARNm codant pour tPa, dans l'échantillon biologique obtenu dans l'étape (b),
dans laquelle le résultat est indicatif d'une réponse positive si les niveaux d'expression de protéine tPA, ou d'ARNm codant pour tPA, dans ledit échantillon biologique sont accrus en comparaison avec un échantillon de référence et/ou un témoin positif.

3. Méthode selon la revendication 3, dans laquelle l'expression accrue est définie comme un niveau d'expression supérieure à l'expression produite dans un échantillon de référence, de préférence des CSM dans un échantillon dans des conditions de culture normales.

4. Méthode selon la revendication 3, dans laquelle l'expression accrue est définie comme un niveau d'expression égal à deux fois l'expression de protéine tPA ou ARNm codant pour tPA dans des CSM dans des conditions de culture normales.

5. Méthode permettant de pronostiquer ou prédire un événement thrombotique associé au traitement avec des CSM d'un sujet humain souffrant de maladie inflammatoire, en prédisant ou pronostiquant la réponse dudit sujet humain audit traitement, dans laquelle ladite méthode comprend :
a) l'obtention d'un échantillon de CSM isolées,
b) le contact des CSM isolées de l'étape (a) avec du sérum sanguin humain obtenu par centrifugation de sang humain du sujet humain,
c) utilisation, comme indicateur, de niveaux d'expression de protéine d'inhibiteur de l'activateur de plasminogène (PAI-1), ou d'ARNm codant pour tPa, dans l'échantillon biologique obtenu dans l'étape (b),
dans laquelle le résultat est indicatif d'une réponse positive si les niveaux d'expression de PAI-1, ou d'ARNm codant pour PAI-1 sont réduits en comparaison avec un échantillon de référence et/ou un témoin positif.

6. Méthode selon la revendication 5, dans laquelle l'expression réduite est définie comme un niveau d'expression inférieure à l'expression produite dans un échantillon de référence, de préférence des CSM dans un échantillon dans des conditions de culture normales.

7. Méthode selon la revendication 5, dans laquelle l'expression réduite est définie comme un niveau d'expression inférieur ou égal à la moitié de l'expression de protéine PAI-1 ou ARNm codant pour PAI-1 dans des CSM dans des conditions de culture normales.

8. Méthode permettant de pronostiquer ou prédire un événement thrombotique associé au traitement avec des CSM d'un sujet humain souffrant de maladie inflammatoire, en prédisant ou pronostiquant la réponse dudit sujet humain audit traitement, dans laquelle ladite méthode comprend :
a) l'obtention d'un échantillon de CSM isolées,
b) le contact des CSM isolées de l'étape (a) avec du sérum sanguin humain obtenu par centrifugation de sang humain du sujet humain,
c) utilisation, comme indicateur, de niveaux d'expression de protéine de l'activateur tissulaire de plasminogène (tPA) ou d'ARNm codant pour tPa, et de protéine d'inhibiteur de l'activateur de plasminogène (PAI-1) ou d'ARNm codant pour PAI-1 dans l'échantillon biologique obtenu dans l'étape (b),
dans laquelle le résultat est indicatif d'une réponse positive si les niveaux d'expression de protéine tPA ou d' ARNm codant pour tPA, dans ledit échantillon biologique, sont réduits en comparaison avec un échantillon de référence et/ou un témoin positif, et si les niveaux d'expression de protéine PAI-1 ou d' ARNm codant pour PAI-1 sont accrus en comparaison avec un échantillon de référence et/ou un témoin positif.

9. Méthode selon la revendication 8, dans laquelle l'expression réduite est définie comme un niveau d'expression inférieure à l'expression produite dans un échantillon de référence, de préférence des CSM dans un échantillon dans des conditions de culture normales et dans laquelle l'expression accrue est définit comme un niveau d'expression supérieur à l'expression produite dans un échantillon de référence, de préférence des CSM dans un échantillon dans des conditions de culture normales.

10. Méthode selon la revendication 8, dans laquelle l'expression réduite est définie comme un niveau d'expression inférieur ou égal à la moitié de l'expression de protéine PAI-1 ou ARNm codant pour PAI-1 dans des CSM dans des conditions de culture normales, et dans laquelle l'expression accrue est définie comme un niveau d'expression supérieur ou égal à deux fois l'expression de protéine tPA ou ARNm codant pour PAI-1 dans des CSM dans des conditions de culture normales.

11. Méthode selon l'une quelconque des revendications 1-10-4, dans laquelle la maladie inflammatoire est le diabète de type 2.

12. Méthode selon l'une quelconque des revendications 1-10-4, dans laquelle la maladie inflammatoire est la maladie vasculaire périphérique.

13. Méthode selon l'une quelconque des revendications 1-10-4, dans laquelle la maladie vasculaire périphérique est une ischémie critique des membres inférieurs.

14. Méthode permettant d'assigner un sujet humain souffrant d'une maladie inflammatoire à un de deux groupes, dans laquelle le groupe 1 comprend des sujets identifiables par le méthode selon l'une quelconque des revendications 1-10 comme sujets humains avec une réponse positive ; et dans laquelle le groupe 2 représente le reste des sujets.
